# EUROPEAN PATENT SPECIFICATION

(11) **EP 1 392 722 B1**
(45) Date of publication and mention of the grant of the patent: **08.04.2009**
(21) Application number: 02725776.5
(22) Date of filing: 08.04.2002
(51) Int. Cl.: C07K 7/56, A61K 38/12

(54) **ECHINOCANDIN PROCESS**
ECHINOCANDINVERFAHREN
PROCEDE VISANT LES ECHINOCANDINES

(30) Priority: 12.04.2001 US 283297 P
(43) Date of publication of application: 03.03.2004
(73) Proprietor: Merck & Co., Inc., Rahway, New Jersey 07065-0907 (US)
(72) Inventor: BELYK, Kevin, M., Rahway, NJ 07065-0907 (US); LEONARD, William, R., Jr., Rahway, NJ 07065-0907 (US)
(74) Representative: Horgan, James Michael Frederic
(86) International application number: PCT/US2002/012756
(87) International publication number: WO 2002/083713

(56) References cited:
- WO-A-00/12540
- US-A- 5 378 804
- US-A- 5 552 521
- US-A- 5 936 062
- US-A- 5 939 384
- MAETZ P ET AL: "A Simple Preparation of N-Protected Chiral alpha-Aminonitriles from N-Protected alpha-Amino Acid Amides" TETRAHEDRON LETTERS, ELSEVIER, AMSTERDAM, NL, vol. 38, no. 24, 16 June 1997 (1997-06-16), pages 4221-4222, XP004074795 ISSN: 0040-4039
- JOURNET ET AL.: 'Semisynthesis of an antifungal lipopeptide echinocandin' J. ORG. CHEM. vol. 64, 1999, pages 2411 - 2417, XP002956497

## Description

### BACKGROUND OF THE INVENTION

This invention relates to an improved process for the minimization of acid-catalyzed reactions of certain echinocandins of the kind disclosed in U.S. Patent No. 5,378,804. The echinocandin compounds disclosed in the patent have been prepared as described in the patent and in patents claiming improvements of the process. U.S. Patent No. 5,552,521 discloses a three-step process for preparing the compounds of the invention. U.S. Patent No. 5,936,062 discloses an improvement of the three-step process using a boronate intermediate. Articles in the Journal of Organic Chemistry, 1999, 64, 2411-2417 and J. Med. Chem. 1994, 37, 222-225, describe an amide to nitrile dehydration of similar echinocandins using cyanuric chloride. However, the previous processes resulted in the formation of unwanted benzylic substituted derivatives of the desired compound. The instant invention results in increased yield of the desired product while minimizing the acid-catalyzed reaction at the benzylic center as well as acid-catalyzed epimerization at the benzylic center. Additionally, the process allows for the use of moderate to strong acid catalysts previously determined to be ineffective at catalyzing the reaction in a highly chemo-selective manner. The improvement permits the use of considerably less acid to complete the formation of the desired compound. Additionally, the α/β stereoselectivity for the phenylsulfide formation reaction is greatly increased.

### SUMMARY OF THE INVENTION

This invention is directed to a process for the minimization of certain impurities generated by acid-catalyzed reactions of certain echinocandins. In particular, the invention describes an improvement to the process of preparing certain sulfide-substituted echinocandins formed as intermediates in the preparation of the compound of the structure

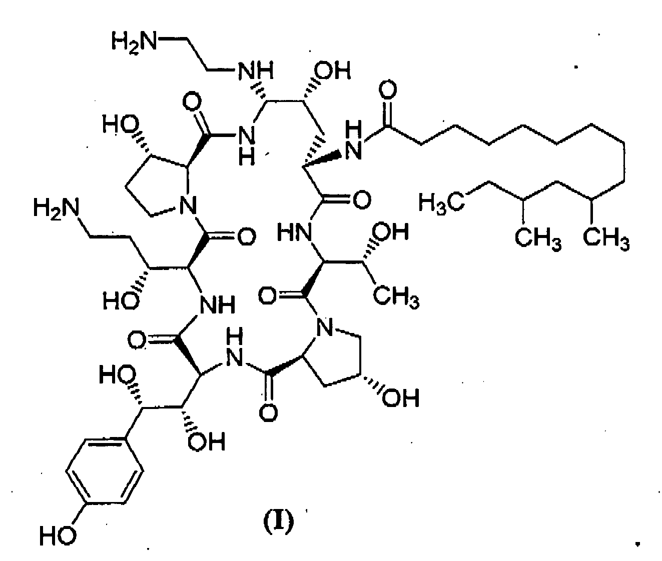

This compound has been found useful in treating life-threatening fungal infections.

### DETAILED DESCRIPTION OF THE INVENTION

The invention discloses a process for preparing Compound 5A of the structure 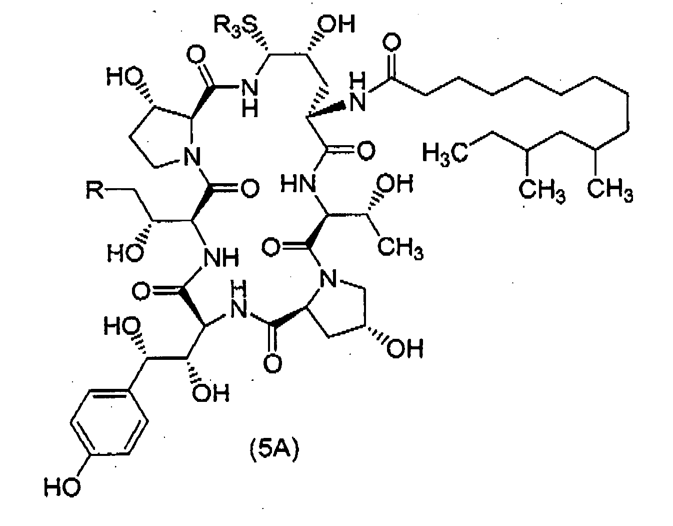 or pharmaceutically acceptable salt, hydrate or solvate thereof, while minimizing the formation of Compounds 5B and Compound 5C of the structure 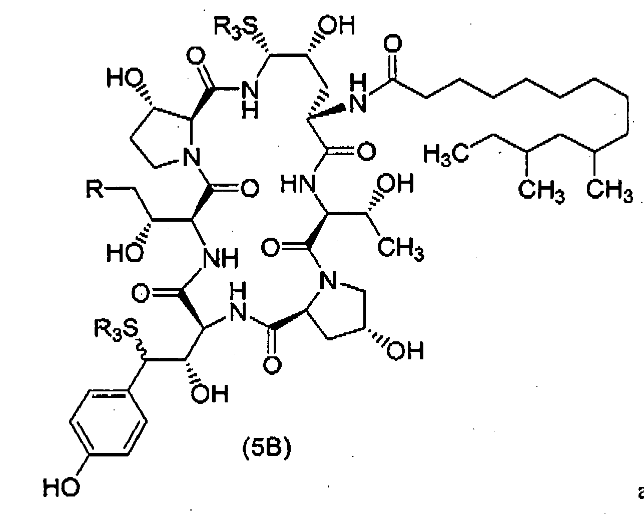 and 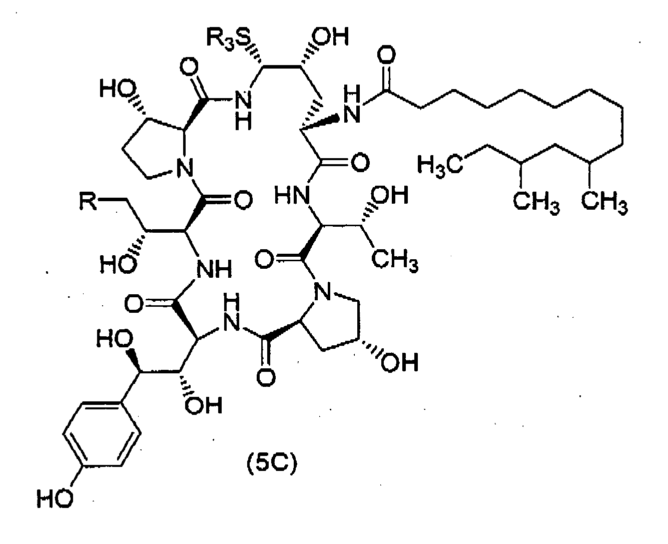 wherein
R is C(=O)NH₂, CH₂NH₃⁺X⁻ or CN;
X- is Cl⁻, CF₃COO⁻, CH₃COO⁻, CF₃SO₃⁻, HSO₄⁻; and
R₃ is aryl, wherein aryl is defined as phenyl or naphthyl, substituted or unsubstituted with C₁-C₆-alkyl, C₁-C₆-alkoxy, halo (Br, Cl, F, I), (CH₂)aryl, heteroaryl, wherein heteroaryl is defined as 5-membered ring, 6-membered ring, 5,6-fused ring, or 6,6-fused ring bearing 1 to 3 heteroatoms selected from N, O, or S optionally substituted with C₁-C₆-alkyl, or C₁-C₆-alkoxy;
which comprises the steps of:
A) reacting Compound 4 of the structure 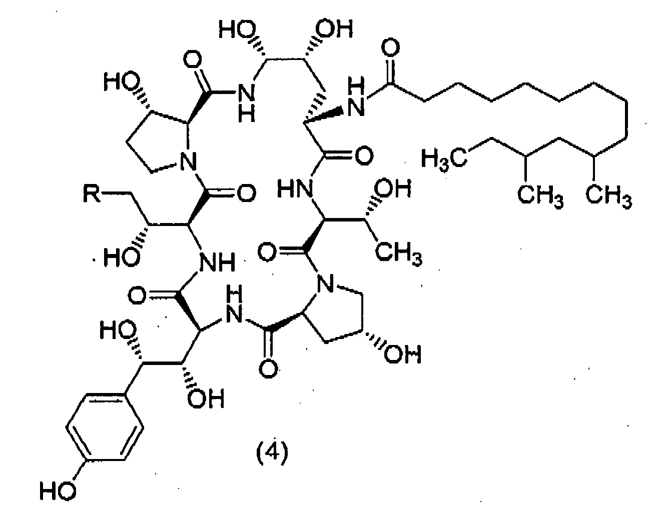 with a boronic acid or borate in a solvent to afford boronate(s) or borate(s), respectively, of compound 4; and
B) reacting the boronate or borate with a thiol, R₃SH, wherein R₃ is as defined above, and an acid to afford Compound 5A with minimal amounts of Compounds 5B and 5C.

In this invention, there is disclosed an improved process for minimizing the acid-catalyzed reaction of R³-SH at the benzylic center which results in a significantly lower formation of the undesired benzylic-substituted bissulfidc derivative, 5B. Prior processes resulted in > 9% of the undesired benzylic-substituted bis(phenylsulfide) derivatives, 5B (wherein R³-SH is thiophenol). This process, wherein R³-SH is thiophenol, also results in an increase in chemical yield to about 92% to about 95% with the formation of only about 2-3% of the undesired benzylic-substituted bis(phenylsulfide) derivatives. The improved process allows for the use of strong acid catalysts previously determined to be ineffective due to excessive substitution or epimerization of the benzylic hydroxyl group. In addition, the improved process allows for the use of significantly less acid to complete the sulfide formation. The sulfide compound, 5A is a key intermediate in the preparation of the compound of Structure I.

The sulfide, Compound 5A is prepared by formation of the boronate/borate in Step A which comprises reaction of Compound 4 (Compound 2, R= C(=O)NH₂; Compound 1, R=CN or Compound 6, R=CH₂NH₃⁺X⁻) with a boronic acid, R₁B(OH)₂, or borate, (R₂O)₃B, in a solvent; followed by reaction of the boronate/borate produced in Step A with a thiol, R₃SH, and an acid, R₄-H⁺, in Step B to produce the sulfide, Compound 5A.

Solvent(s) such as tetrahydrofuran, acetonitrile, or mixtures thereof can be used in reaction Step A. Any boronic acid or borate is expected to produce the desired results. Examples of such boronic acid or borate include, but are not limited to, phenylboronic acid, 3-nitrophenylboronic acid, 3,5-bis(trifluoromethyl)phenylboronic acid, 4-methoxyphenylboronic acid, *n*-butylboronic acid, trimethyl borate and triethyl borate. The reaction is carried out at a temperature range of about 20°C to about 66°C and for a reaction time of about 15 minutes to about 12 hours.

A preferred embodiment of this invention is the process Step A as recited above, wherein the boronic acid is phenylboronic acid and the solvent is CH₃CN.

Following the formation of the boronate/borate in Step A, the boronate/borate product is combined with a thiol and suitable acid in Step B to yield the desired sulfide, Compound 5A in a highly stcrcoselective and chemoselective manner. Any thiol is expected to produce the sulfide intermediate. R₄⁻H⁺ represents a suitable acid, a moderate to strong acid that would be expected to produce the sulfide intermediate in good yield. Examples of such moderate to strong acids include, but are not limited to, trifluoroacetic acid, methanesulfonic acid, p-toluenesulfonic acid, and trifluoromethanesulfonic acid.

A preferred embodiment of this invention is the process Step A as recited above, wherein the thiol is thiophenol and the R₄⁻H⁺ is trifluoromethanesulfonic acid.

When reaction Step B is complete, the mixture is quenched with water or dilute base and, following an annealing step, the sulfide intermediates are isolated by filtration.

The amount of boronic acid or borate used is crucial to the rate of displacement, as well as to the formation of the undesirable bis(sulfide) derivative (Compound 5B) and benzylic hydroxyl epimer (Compound 5C). It was found that from about 100 to about 900 mole % of a boronic acid or a borate gave the best yield of Compound 5A with about 100 to about 300 mole % of the boronic acid being the preferred range.

The amount of thiol used in the sulfide formation step is also critical to the yield of Compound 5A. About 200 to about 500 mole % of thiophenol provided the best yield of the phenylsulfide intermediate (Compound 5A).

The amount of R₄⁻H⁺ used is also critical to the yield of Compounds 5A and formation of the undesired Compounds 5B and 5C. It was found that about 200 to about 4600 mole % of a moderate to strong acid catalyst gave the best yield. Additionally, the conditions provide optimal process aging time, chemoselectivity, stereoselectivity, and the best means of producing an easily filtered product.

A preferred embodiment of the invention is the process as recited above wherein Compound 4 is reacted with about 200 mole % of phenylboronic acid in acetonitrile at ambient temperature for about 0.5 hours followed by reaction of the boronate with 300 mole % thiophenol and about 300 mole % trifluoromethanesulfonic acid at about -13°C. HPLC analysis showed a chemical yield of about 95% with concomitant formation of about 3 area % of the bis(phenylsulfide) and 0.1 area % of the benzylic hydroxyl epimer, Compounds 5B and 5C respectively. The reaction mixture is diluted to 90% acetonitrile/10% water (v/v) with water containing 300 mole % sodium acetate. The product was isolated by filtration after an annealing step. The phenylsulfide intermediate was isolated in about 91 % yield and contained 1.1 area % of the undesired benzylic-substituted bis(phenylsulfide) derivative (Compound 5B) and <0.1 area % of the undesired benzylic hydroxyl epimer (Compound 5C) as analyzed by HPLC.

An additional embodiment of the process for preparing the sulfide Compound 5A, is the process which includes the additional steps of:
(i) quenching the reaction mixture containing Compound 5A with aqueous base and cooling the reaction mixture to precipitate Compound 5A as a suspension of fine amorphous particles of Compound 5A;
(ii) warming the suspension of Compound 5A to about 15 to about 25°C over about 1.5 to 2.0 hours to produce larger amorphous particles of Compound 5A and aging the suspension of Compound 5A for about 20 to about 30 minutes at about 15 to about 25°C;
(iii) cooling the suspension with larger amorphous particles of Compound 5A to 0°C and aging the suspension of Compound 5A for about 1 hour; and
(iv) filtering the suspension of larger amorphous particles of Compound 5A to isolate larger amorphous particles of Compound 5A.

The Compound 4, wherein R represents CH₂NH₃⁺X⁻ (also referred to as Compound 6) is prepared by chemical or catalytic reduction of the nitrile compound, Compound 1. Chemical reduction can be carried out using sodium borohydride, aluminum hydride, diborane, diisobutyl aluminum hydride and the like. Catalytic reduction may also be employed using hydrogenation with a variety of catalysts including palladium on carbon, platinum oxide, or rodium on alumina. See US Patent 5,939,384 and the Journal of Organic Chemistry, 1999, 64, 2411-2417.

A process for the preparation of a Compound 1 of structure 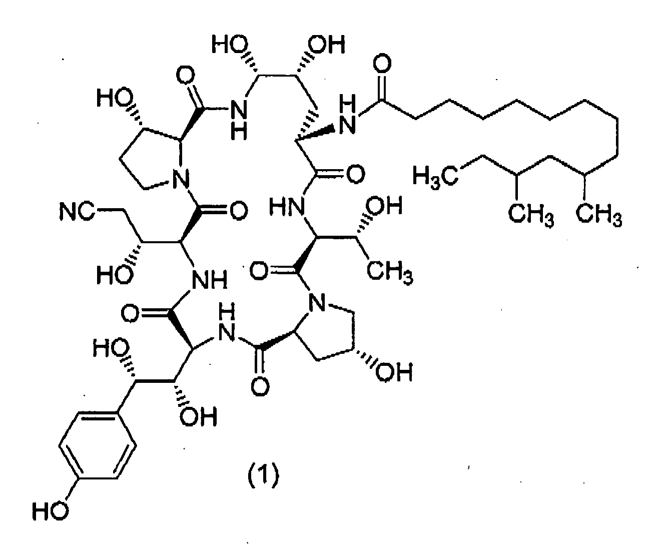 or its pharmaceutically acceptable salt, hydrate or solvate thereof, comprises the steps of:
a) reacting an amide Compound 2 of the structure 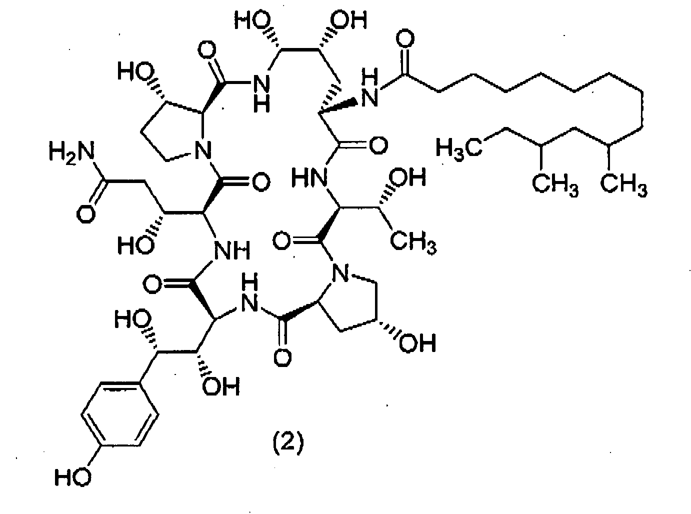 with a boronic acid or borate in a solvent to obtain a boronate or borate, respectively; and
b) dehydrating the boronate or borate with cyanuric chloride in a second solvent to produce a nitrile Compound 1 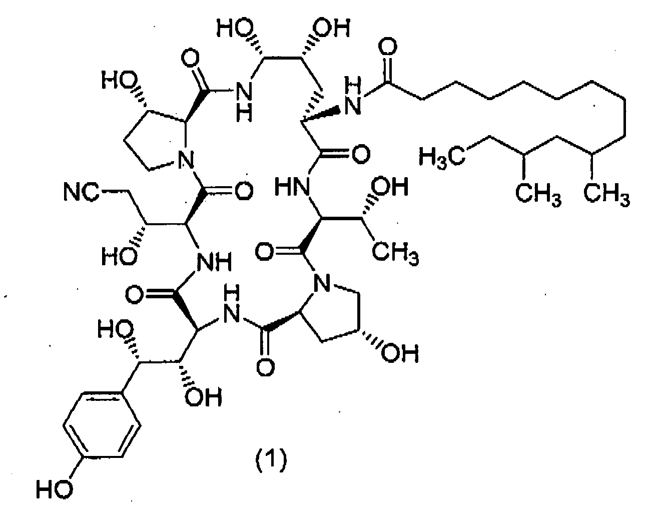 with concomitant formation of a minimal amount of an epimer of the nitrile Compound 1, Compound 3 having the structure

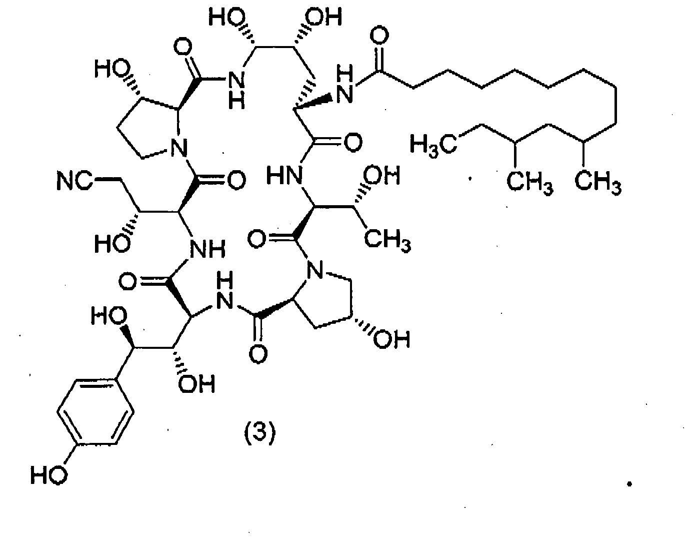

Compound 2 can be produced by cultivating *Glarea lozoyensis* (formerly identified as *Zalerion arboricola*) ATCC 20868 in a nutrient medium enriched in mannitol as the primary source of carbon, as described in US Patent No. 5,021,341, issued June 4,1991. Compound 2 is claimed in US Patent No. 5,202,309 that issued April 13, 1993. Compound 2 can also be produced by cultivating *Zalerion arboricola* in a nutrient medium enriched the unnatural amino acid selected from the group consisting of: 3,4-dehydroproline, L-azetidine-2-carboxylic acid, L-proline or mixture, as described in US Patent No. 5,194,377 issued on March 16,1993.

Step a involves reaction of Compound 2 and a boronic acid in a first solvent. About 100 to about 200 mole % of the boronic acid can be employed, and about 200 mole % of the boronic acid is preferred. Any boronic acid or borate can be used to produce the desired result. Examples of such boronic acids or borates arc: phenylboronic acid, 3-nitrophenylboronic acid, 4-methoxyphenylboronie acid, 3,5-bis(trifluoromethyl)phenylboronic acid, n-butylboronic acid, trimethyl borate and triethylborate. A preferred boronic acid is phenyl boronic acid. Examples of such first solvent are tetrahydrofuran, CH3CN or a mixture therefrom.

When formation of the borte/boronate is complete in Step a, the water is removed from the reaction mixture. The water is removed by azeotropic distillation of the reaction solvent with concomitant addition of dry solvent or by passing the refluxate through a bed of molecular sieves until a ratio of less than about 20 mole % water to Compound 2 is obtained.

After removal of the water from the reaction mixture, the solvent is removed *in vacuo* and subsequent dissolution of the borate/boronate derivative with a second solvent. Suitable second solvent(s) such as dry *N*,*N-*dimethylformamide (DMF), tetrahydrofuran , or *N*-methylpyrrolidinone (NMP) or mixtures thereof can be used. The preferred solvent is a mixture of *N*-methylpyrrolidinone and *N*,*N-*dimethylformamide.

Step b involves dehydration of the borate/boronate derivative in the suitable second solvent with cyanuric chloride or other appropriate reagent to yield the nitrile. The amount of cyanuric chloride used is crucial to limit the formation of the undesired hydroxyl epimer at the benzylic position of the homo-tyrosine portion of the cyclic peptide. About 100 to about 300 mole % of cyanuric chloride can be employed, and about 180 to about 230 mole % is the preferred range.

Preferably about 200 mole % phenylboronic acid in tetrahydrofuran is used in Step a at ambient temperature, followed by removal of water by azeotropic distillation of the refluxate through molecular sieves (3A). The reaction mixture is then dried and the solvent removed *in vacuo.* The resulting solids were dissolved in the second solvent(s) a 9:1 *N*-methylpyrrolidinone/*N,N*-dimethylformamide volume to volume mixture and dehydrated with 230 mole % of cyanuric chloride. The cyanuric chloride was added at-13°C and the reaction mixture was aged at -13°C to -23°C for 20 hours. These conditions resulted in a chemical yield of about 84% with no benzylic hydroxyl epimer (Compound 3) detected by HPLC analysis.

The invention is described in greater detail in the following examples in which all parts, preparations, ratios and percentages are by weight unless otherwise indicated.

### EXAMPLE 1

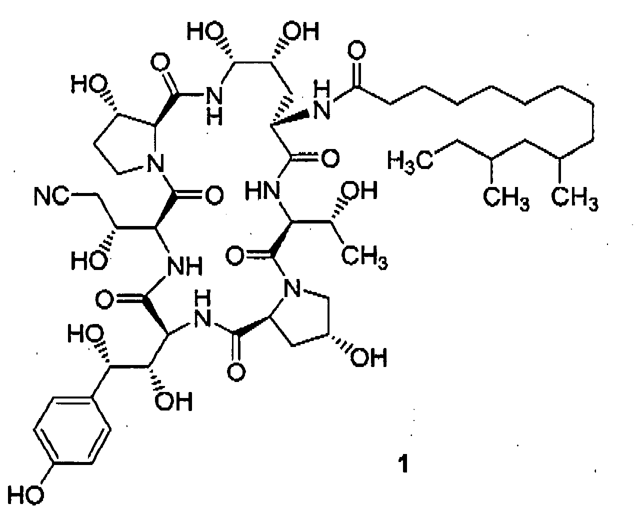

### Synthesis of Compound 1

The amide, Compound 2, (1.0 g, 0.94 mmole) and phenylboronic acid (235 mg, 1.90 mmole) were added to dry tetrahydrofuran (10 mL) and the suspension was agitated until all solids dissolved (40 min). The resulting solution was dried to <20 mol% water: Compound 2 by passing the refluxate through a bed of molecular sieves (3A). A portion of the dried solution (1.0 mL) containing Compound 2 boronate (120 mg, 0.12 mmole) was transferred to a 25 mL flask where the tetrahydrofuran was removed under vacuum. The resulting solid was dissolved in 4.0 mL dry 1-methyl-2-pyrrolidinone and 0.45 mL dry *N,N*-dimethylformamide at ambient temperature. Cyanuric chloride (50 mg, 0.27 mmole) was then added at-13°C with stirring. The reaction was aged for 2.5 hours at -13°C followed by 18 hours at -23°C. HPLC analysis (210 nm) showed 3 area % Compound 2 and an 84% yield of Compound 1. Compound 3 was not detected by HPLC at this time nor was it observed after an additional 26 hours of the reaction at -20°C.

### EXAMPLE 2

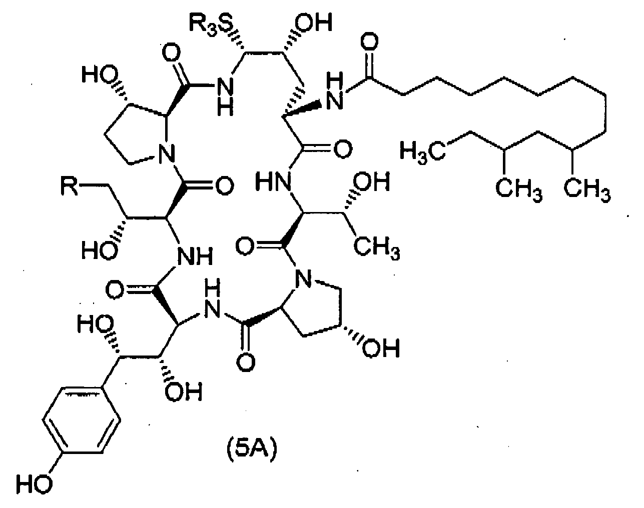

### Synthesis and Isolation of Compound 5A (R= C(=O)NH₂, R₃=phenyl)

Compound 4 (R=C(=O)NH₂, 23.1 g, 21.7 mmole) and phenylboronic acid (5.72 g, 46.9 mmole) were added to dry acetonitrile (750 mL). The suspension was stirred at ambient temperature for 0.5 hours and then cooled to -6°C where thiophenol (7.24 mL, 70.4 mmole) was added. Trifluoromethanesulfonic acid (6.23 mL, 70.4 mmole) was then added over 10 minutes while maintaining a temperature of -13°C. The reaction progress was monitored by HPLC until the ratio of starting maternal/ product was 1:99 (2 hours). The chemical yield of phenylsulfide intermediate for the reaction was 95% as a 60:1 mixture of α- and β-diastereomers by HPLC assay. The undesired benzylic-substituted bis(phenylsulfide) derivative (Compound 5B, R=C(=O)NH₂, R₃=phenyl) and the undesired benzylic hydroxyl epimer (Compound 5C, R=C(=O)NH₂, R₃=phenyl) were present at 3.2 HPLC area % (210 nm) and 0.1 HPLC area % (210 nm), respectively. At 2.5 hours, a solution of NaOAc-3H₂O (9.58 g, 70.4 mmole) in 80 mL water was added at a rate so as to maintain the temperature below -9°C. The product precipitated as 1-2 µm amorphous particles. The suspension was warmed to 19°C over 1.25 hours during which the amorphous precipitate turned over to 5-25 µm amorphous particles. The suspension was then aged 20 minutes at 19°C and then cooled to 0°C and aged 1 hour. The solids were removed by filtration and washed with 3 x 100 mL of 9:1 acetonitrile:water (v/v). The material was then dried under a nitrogen flow. The assay yield of the phenylsulfide was 22.7 g (91%) as a 180:1 mixture of α- and β- diastereomers. The solid contained 1.1 HPLC area % (210 nm) of the undesired benzylic-substituted bis(phenylsulfide) derivative (Compound 5B, R=C(-O)NH₂, R₃=phenyl) and <0.1 HPLC area % (210 nm) of the undesired benzylic hydroxyl epimer (Compound 5C, R-C(=O)NH₂, R₃=phenyl) by HPLC analysis.

## Claims

1. A process for preparing Compound 5A of the structure 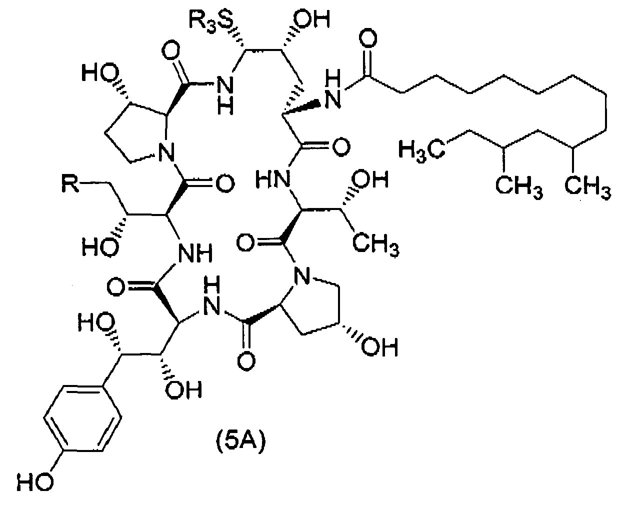 while minimizing the formation of Compounds 5B and Compound 5C of the structure 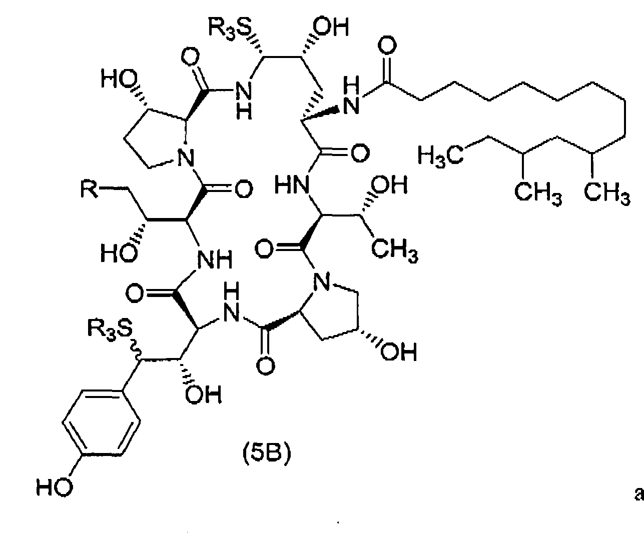 and 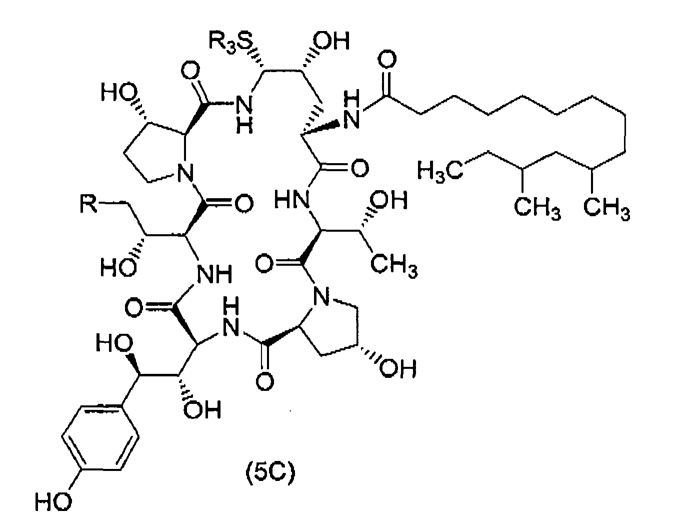 or their pharmaceutically acceptable salts, hydrates or solvatcs thereof, which comprises the steps of:
A) reacting Compound 4 of the structure 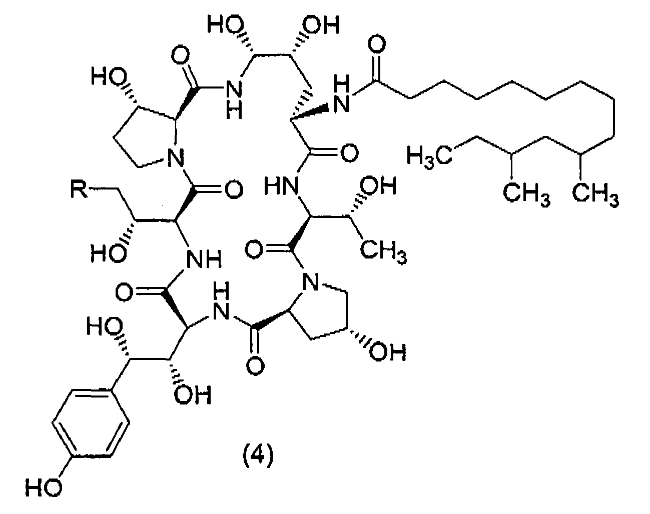 wherein
R is C(=O)NH₂, CH₂NH₃⁺X⁻ or CN;
X- is Cl⁻, CF₃COO⁻, CH₃COO⁻, CF₃SO₃⁻, HSO₄⁻; and
R₃ is aryl, wherein aryl is defined as phenyl or naphthyl, substituted or unsubstituted with C₁-C₆-alkyl, C₁-C₆-alkoxy, halo (Br, Cl, F, I), (CH₂)aryl, heteroaryl, wherein heteroaryl is defined as 5-membered ring, 6-membered ring, 5,6-fussed ring, or 6,6-fused ring bearing 1 to 3 heteroatoms selected from N, O, or S optionally substituted with C₁-C₆-alkyl, or C₁-C₆-alkoxy;
with a boronic acid or borate in a solvent to afford boronate(s) or borate(s), respectively, of compound 4; and
B) reacting the boronate or boratc with a thiol, R₃SH, wherein R₃ is as defined above, and an acid to afford Compound 5A with minimal amounts of Compounds 5B and 5C.

2. The process of Claim 1, wherein the boronic acid or borate in Step A is selected from the group consisting of: aryl boronic acid, wherein aryl is defined as phenyl, 1- or 2-naphthyl, 9-anthryl or phenanthryl, unsubstituted or substituted with 1, 2 or 3 substitutents selected from the group consisting of: nitro, C₁-C₆-alkyl, C₁-C₆-alkoxy, halo (Br, Cl, F, I), and trifluoromethyl, C₁-C₆-alkyl boronic acid, or tri(C₁-C₆-alkyl) borate.

3. The process of Claim 1 or Claim 2, wherein the solvent used in Step A is tetrahydrofuran, CH₃CN or a mixture therefrom.

4. The process of any one of Claims 1 to 3, wherein the acid used in Step B is selected from the group consisting of: trifluoroacetic acid, methanesulfonic acid, p-toluenesulfonic acid, and trifluoromethanesulfonic acid.

5. The process of Claim 4, wherein the boronic acid used in Step A is phenylboronic acid and the solvent used in Step A is CH₃CN.

6. The process of any one of Claims 1 to 5, which includes the additional steps of:
(i) quenching the reaction mixture containing Compound 5A with aqueous base and cooling the reaction mixture to precipitate Compound 5A as a suspension of fine amorphous particles of Compound 5A;
(ii) warming the suspension of compound 5A to 15 to 25°C over 1.5 to 2.0 hours to produce larger amorphous particles of Compound 5A and aging the suspension of Compound 5A for 20 to 30 minutes at 15 to 25°C;
(iii) cooling the suspension with larger amorphous particles of Compound 5A to 0°C and aging the suspension of Compound 5A for about 1 hour; and
(iv) filtering the suspension of larger amorphous particles of Compound 5A to isolate larger amorphous particles of Compound 5A.

## Patentansprüche

1. Ein Verfahren zur Herstellung von Verbindung 5A der Struktur 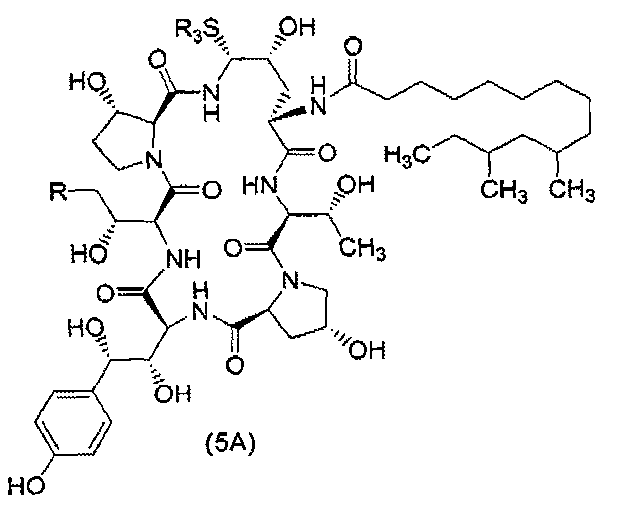 unter Minimierung der Bildung der Verbindungen 5B und Verbindung 5C der Struktur 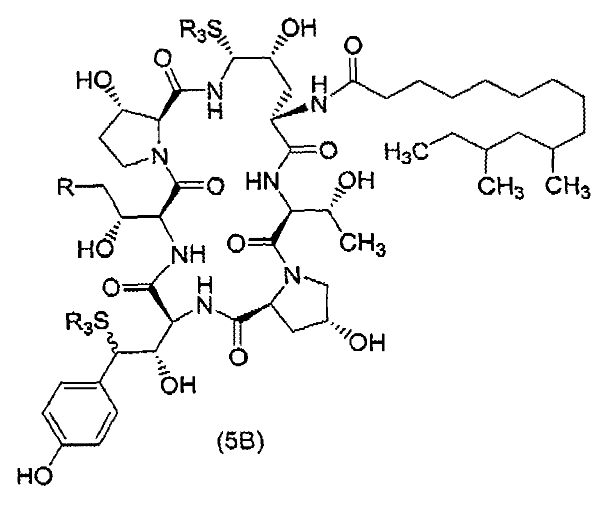 und 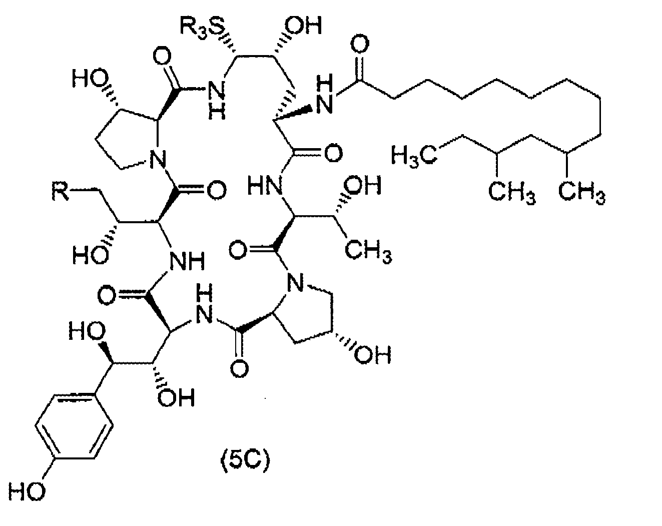 oder deren pharmazeutisch annehmbaren Salzen, Hydraten oder Solvaten davon, umfassend die Schritte:
A) Umsetzung von Verbindung 4 der Struktur 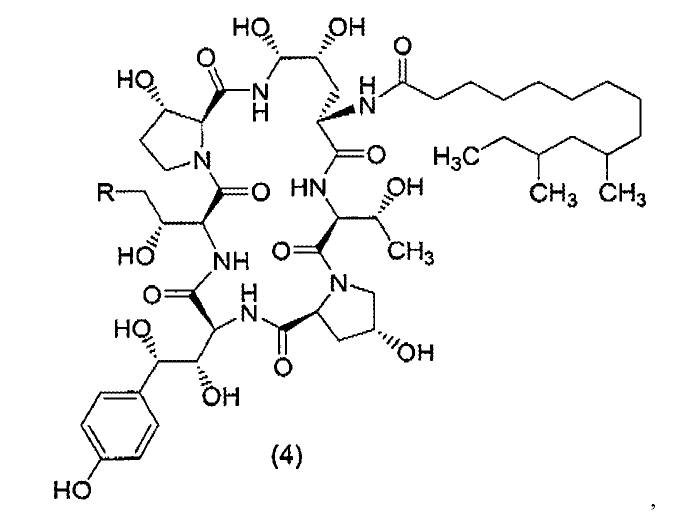 wobei
R C(=O)NH₂, CH₂NH₃⁺X⁻ oder CN ist,
X⁻ Cl⁻, CF₃COO⁻, CH₃COO⁻, CF₃SO₃⁻, HSO₄⁻ ist und
R₃ ist Aryl, wobei Aryl definiert ist als Phenyl oder Naphthyl, substituiert oder unsubstituiert mit C₁-C₆-Alkyl, C₁-C₆-Alkoxy, Halogen (Br, Cl, F, I), (CH₂)Aryl, Heteroaryl, wobei Heteroaryl definiert ist als 5-gliedriger Ring, 6-gliedriger Ring, 5,6-kondensierter Ring oder 6,6-kondensierter Ring, der 1 bis 3 Heteroatome, ausgewählt aus N, O oder S, trägt, gegebenenfalls substituiert mit C₁-C₆-Alkyl oder C₁-C₆-Alkoxy,
mit einer Boronsäure oder einem Borat in einem Lösungsmittel, um Boronat(e) bzw. Borat(e) von Verbindung 4 zu ergeben, und
B) Umsetzung des Boronats oder Borats mit einem Thiol, R₃SH, wobei R₃ wie oben definiert ist, und einer Säure, um Verbindung 5A mit minimalen Mengen der Verbindungen 5B und 5C zu ergeben.

2. Das Verfahren nach Anspruch 1, wobei die Boronsäure oder das Borat in Schritt A ausgewählt ist aus der Gruppe, bestehend aus: Arylboronsäure, wobei Aryl definiert ist als Phenyl, 1- oder 2-Naphthyl, 9-Anthryl oder Phenanthryl, unsubstituiert oder substituiert mit 1, 2 oder 3 Substituenten, ausgewählt aus der Gruppe, bestehend aus: Nitro, C₁-C₆-Alkyl, C₁-C₆-Alkoxy, Halogen (Br, Cl, F, I) und Trifluormethyl, C₁-C₆-Alkylboronsäure oder Tri(C₁-C₆-alkyl)borat.

3. Das Verfahren nach Anspruch 1 oder Anspruch 2, wobei das in Schritt A verwendete Lösungsmittel Tetrahydrofuran, CH₃CN oder eine Mischung davon ist.

4. Das Verfahren nach irgendeinem der Ansprüche 1 bis 3, wobei die in Schritt B verwendete Säure ausgewählt ist aus der Gruppe, bestehend aus: Trifluoressigsäure, Methansulfonsäure, p-Toluolsulfonsäure und Trifluormethansulfonsäure.

5. Das Verfahren nach Anspruch 4, wobei die in Schritt A verwendete Boronsäure Phenylboronsäure ist und das in Schritt A verwendete Lösungsmittel CH₃CN ist.

6. Das Verfahren nach irgendeinem der Ansprüche 1 bis 5, umfassend die zusätzlichen Schritte:
(i) Quenchen der Reaktionsmischung, welche Verbindung 5A enthält, mit wässriger Base und Abkühlen der Reaktionsmischung, um Verbindung 5A als eine Suspension von feinteiligen amorphen Teilchen von Verbindung 5A abzuscheiden,
(ii) Erwärmen der Suspension von Verbindung 5A auf 15 bis 25°C innerhalb von 1,5 bis 2,0 Stunden, um größere amorphe Teilchen von Verbindung 5A zu erzeugen, und 20- bis 30-minütiges Altern der Suspension von Verbindung 5A bei 15 bis 25°C.
(iii) Abkühlen der Suspension mit größeren amorphen Teilchen von Verbindung 5A auf 0°C und etwa 1-stündiges Altern der Suspension von Verbindung 5A, und
(iv) Filtrieren der Suspension von größeren amorphen Teilchen von Verbindung 5A, um größere amorphe Teilchen von Verbindung 5A zu isolieren.

## Revendications

1. Procédé pour la préparation du composé 5A de la structure: 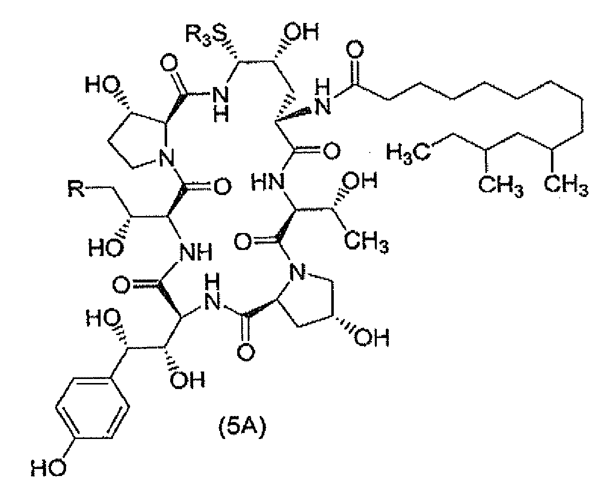 tout en minimisant la formation du composé 5B et du composé 5C des structures: 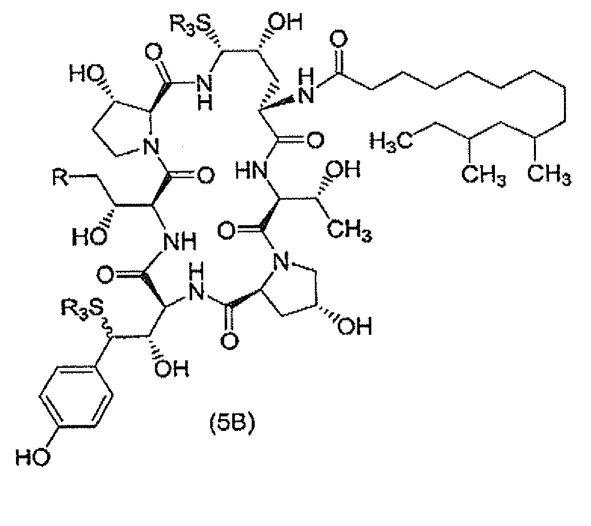 et 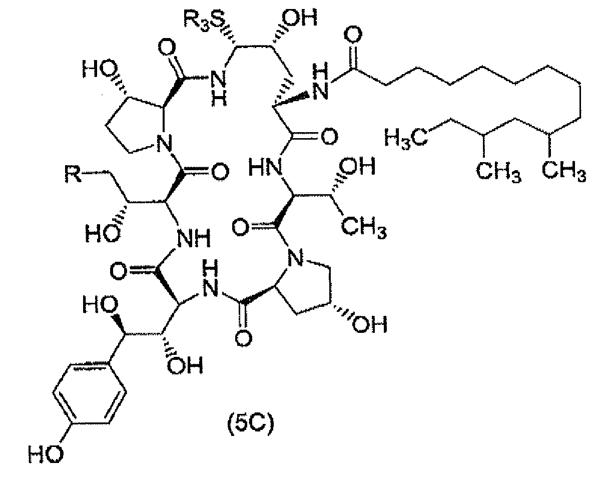 ou de leurs sels pharmaceutiquement acceptables, hydrates ou solvates de ceux-ci, qui comprend les étapes:
A) de réaction du composé 4 de la structure: 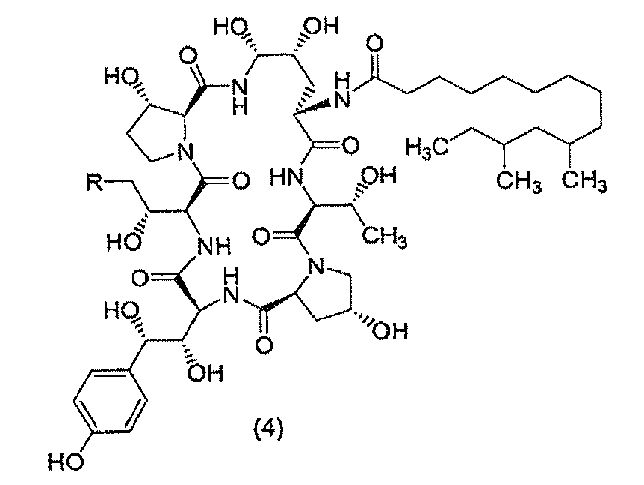 dans laquelle:
R est C(=O)NH₂, CH₂NH₃⁺X⁻ ou CN;
X⁻ est Cl⁻, CF₃COO⁻, CH₃COO⁻, CF₃SO₃⁻, HSO₄⁻; et
R₃ est un groupe aryle, où le groupe aryle est défini comme un groupe phényle ou naphtyle, non substitué ou substitué avec un groupe alkyle C₁-C₆, alcoxy C₁-C₆, halo (Br, Cl, F, I), (CH₂)aryle, hétéroaryle, où le groupe hétéroaryle est défini comme un cycle à 5 membres, un cycle à 6 membres, un cycle 5,6-condensé ou un cycle 6,6-condensé portant de 1 à 3 hétéroatomes choisis parmi N, O ou S éventuellement substitué avec un groupe alkyle C₁-C₆ ou alcoxy C₁-C₆;
avec un acide boronique ou un borate dans un solvant pour donner un ou des boronates ou un ou des borates, respectivement, du composé 4; et
B) de réaction du boronate ou du borate avec un thiol, R₃SH, où R₃ est comme il a été défini ci-dessus, et un acide pour donner le composé 5A avec des quantités minimales des composés 5B et 5C.

2. Procédé selon la revendication 1, dans lequel l'acide boronique ou le borate dans l'étape A est choisi dans le groupe constitué: d'acide arylboronique, où le groupe aryle est défini comme un groupe phényle, 1- ou 2-naphtyle, 9-anthryle ou phénanthryle, non substitué ou substitué avec 1, 2 ou 3 substituants choisis dans le groupe constitué: de groupes nitro, alkyle C₁-C₆, alcoxy C₁-C₆, halo (Br, Cl, F, I) et trifluorométhyle, d'acide (alkyle C₁-C₆)boronique ou de tri(alkyle C₁-C₆)borate.

3. Procédé selon la revendication 1 ou la revendication 2, dans lequel le solvant utilisé dans l'étape A est le tétrahydrofuranne, CH₃CN ou un mélange de ceux-ci.

4. Procédé selon l'une quelconque des revendications 1 à 3, dans lequel l'acide utilisé dans l'étape B est choisi dans le groupe constitué: d'acide trifluoroacétique, d'acide méthanesulfonique, d'acide p-toluènesulfonique et d'acide trifluorométhanesulfonique.

5. Procédé selon la revendication 4, dans lequel l'acide boronique utilisé dans l'étape A est l'acide phénylboronique et le solvant utilisé dans l'étape A est CH₃CN.

6. Procédé selon l'une quelconque des revendications 1 à 5, qui inclut les étapes supplémentaires:
(i) de trempe du mélange de réaction contenant le composé 5A avec une base aqueuse et de refroidissement du mélange de réaction pour précipiter le composé 5A sous forme d'une suspension de particules amorphes fines du composé 5A;
(ii) de réchauffement de la suspension du composé 5A à 15 à 25°C sur une période de 1,5 à 2,0 heures pour produire des particules amorphes de plus grande taille du composé 5A et de vieillissement de la suspension du composé 5A pendant de 20 à 30 minutes à 15 à 25°C;
(iii) de refroidissement de la suspension avec des particules amorphes de plus grande taille du composé 5A à 0°C et de vieillissement de la suspension du composé 5A pendant environ 1 heure; et
(iv) de filtration de la suspension de particules amorphes de plus grande taille du composé 5A pour isoler les particules amorphes de plus grande taille du composé 5A.
